# EUROPEAN PATENT APPLICATION

(11) **EP 4 450 074 A1**
(43) Date of publication of application: **23.10.2024**
(21) Application number: 23315081.2
(22) Date of filing: 18.04.2023
(51) Int. Cl.: A61K 33/00, A61L 24/00, A61L 24/04, A61L 27/18, A61L 27/54, A61L 27/56, A61L 27/58

(54) **COMPOSITION FOR USE IN TISSUE RECONSTRUCTION AND TISSUE REGROWTH**

(71) Applicant: Tissium SA, 75012 Paris (FR)
(72) Inventor: Llusar, Elodie, 93100 Montreuil (FR); Gerbouin, Prune, 75020 Paris (FR); Pereira, Maria, 1200-366 Lisbon (PT); Lopes, Miguel, 2780-143 Oeiras (PT)
(74) Representative: Finnegan Europe LLP

(57) **Abstract**

The present disclosure relates to a composition in the treatment of pathological situations where tissue reconstruction and tissue regrowth are necessary, more particularly for use as a tissue lumen and void filler, e.g. in the treatment of fistulas, wherein the composition comprises a pre-polymeric component and a porogen.

## Description

### FIELD OF INVENTION

The present invention relates to compositions for use in the treatment of pathological situations where tissue reconstruction and tissue regrowth are necessary, more particularly for use as a soft tissue lumen and void filler, e.g. in the treatment of fistulas, and to compositions, syringes, and kits, suitable for use in these treatments. The present invention further relates to methods for the treatment of pathological situations where tissue reconstruction and tissue regrowth are necessary, e.g. treatment of fistulas.

### BACKGROUND OF THE INVENTION

A fistula is an abnormal passage between two organs or vessels that do not usually connect. Perianal fistulas can occur between the anal canal, including the colon, bowel or rectum, and the perianal skin. Other fistulas, named for the areas of the body in which they occur, include enterocutaneous fistulas (between the small bowel and abdominal skin), rectovaginal fistulas (between the rectum and the vagina), and oroantral fistulas (between the oral cavity and sinuses).

Perianal fistulas most commonly develop from an anal abscess or as a consequence of inflammatory bowel diseases (usually Crohn's disease). If left untreated, fistulas can have a high impact on a patient's quality of life, both physical and psychological.

Perianal fistulas are classified based on their morphology, positioning and percentage of external sphincter affected. About 70% are classified as simple, and 30% are classified as complex. Despite a variety of available treatment options for perianal fistulas, none of those currently available have both high success rate and low sphincter damage. Damage to the sphincter can lead to fecal incontinence. Moreover, depending on the morphology of the fistula and if anal tissue is diseased, the patient may not be eligible for all procedures.

Fistulotomy is a surgical procedure wherein the fistula is cut open so that it may heal as a flat scar. Such a procedure is only suitable for simple fistulas. For complex fistulas, surgical techniques include advancement flap surgery and ligation of intersphincteric fistula tract (LIFT) treatment. Device-based treatments for complex fistulas include use of bioprosthetic plugs and fibrin glue injections.

Surgical treatments typically have reasonable success rates but carry a risk of recurrence, reinfection and fecal incontinence. Device-based treatments (plugs and glues) have a lower risk of fecal incontinence but have other limitations such as extrusion of the plug or glue, failure of the device to adequately fill the fistula, or poor usability of glue leading to leakage during the procedure. Therefore, there remains an unmet medical need for a new solution for fistula treatment, and more generally treatment of pathological situations where tissue reconstruction and tissue regrowth are necessary, that can be efficient while preserving the sphincter, being safe and easy to use.

### SUMMARY OF THE INVENTION

The present invention provides a treatment option for pathological situations where tissue reconstruction and tissue regrowth are necessary. More particularly, the present invention provides a treatment option for use as a tissue lumen and void filler, e.g. a treatment option for fistulas, that avoids the disadvantages of prior art treatments. In particular, the present invention provides a composition that will aid patients in the healing and closure of a fistula with lower or no associated risk of incontinence. In particular, the composition i) conforms to the tissue lumen and void filler, e.g. a fistula tract and ii) is not extruded from the body during healing, namely by adhering to tissue and being colonized by cells after implantation. The composition may increase healing rate when compared to existing products, while having less or no impact on continence for example.

The composition may be delivered into targeted localisation, for example into a fistula, e.g. using a syringe. The viscosity of the composition may be such that it is readily delivered, e.g. into the fistula, and its adhesive property once polymerization occurs *in situ* may be such that it is retained in place, e.g. within the fistula. The pre-polymeric component in the composition may undergo cross-linking whilst in place, e.g. in the fistula such that the composition becomes adhesive, leading to better retention of the composition, e.g. within the fistula.

The invention provides a composition for use in pathological situations where tissue reconstruction and tissue regrowth are necessary, more particularly for use as a soft tissue lumen and void filler, and more particularly, for use in treating fistulas, wherein the composition comprises a pre-polymeric component and a porogen;
wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 1.

The invention also provides a composition comprising:
a pre-polymeric component and a porogen;
wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 1, wherein there are functional groups on the polymeric backbone including functional groups of formula (I): 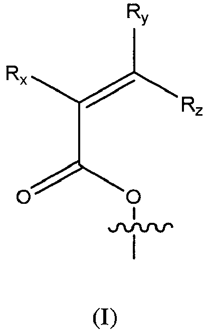
wherein Rₓ, R_{y} and R_{z} are independently selected from H, alkyl, alkenyl and aryl.

According to a preferred embodiment, the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 1, wherein there are functional groups on the polymeric backbone including functional groups of formula (I): 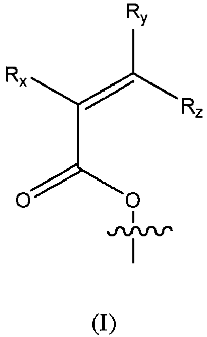 wherein Rₓ, R_{y} and R_{z} are independently selected from H, alkyl, alkenyl and aryl, and further including one or more functional groups that include a charged atom.

According to an embodiment, the composition of the invention further comprises at least one photoinitiator and/or redox initiator system.

The invention also provides a syringe comprising a composition according to the invention.

The invention also provides a kit of parts comprising, formulated separately from one another,
(i) a first composition comprising a pre-polymeric component, wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 1; and
(ii) a second composition comprising a porogen.

According to an embodiment, there are functional groups on the polymeric backbone of the pre-polymeric component of said first composition, including functional groups of formula (I): 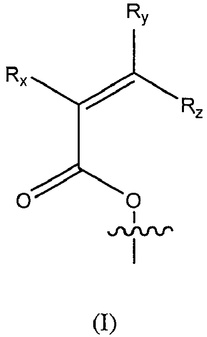 wherein Rₓ, R_{y} and R_{z} are independently selected from H, alkyl, alkenyl and aryl, and optionally the polymeric backbone of the pre-polymeric component further includes one or more functional groups that include a charged atom.

The invention also provides a method for treating pathological situations where soft tissue reconstruction and tissue regrowth are necessary, more particularly for use as a soft tissue lumen and void filler, e.g. in a method for treating fistulas, comprising delivering a composition (e.g. into the fistula), wherein the composition comprises
a pre-polymeric component and a porogen;
wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 1.

According to an embodiment, the polymeric backbone of the said pre-polymeric component includes functional groups of formula (I): 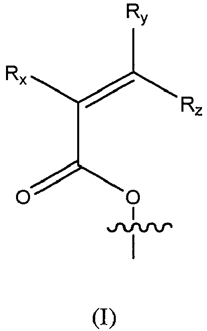 wherein Rₓ, R_{y} and R_{z} are independently selected from H, alkyl, alkenyl and aryl, and optionally the polymeric backbone of the pre-polymeric component further includes one or more functional groups that include a charged atom.

While the invention is particularly described for use in treating fistulas, it is important to note that the present invention can similarly be used in treating any pathological situations where soft tissue reconstruction and tissue regrowth are necessary, more particularly for use as a soft tissue lumen and void filler.

### DETAILED DESCRIPTION OF THE INVENTION

### Pre-polymeric component

The composition comprises a pre-polymeric component having a polymeric backbone with a polymeric unit of general formula (-A-B-)ₙ, wherein A is derived from a substituted or unsubstituted polyol and B is derived from a substituted or unsubstituted polyacid, and n is greater than 1.

The term "substituted" has its usual meaning in chemical nomenclature and is used to describe a chemical compound in which a hydrogen on the primary carbon chain has been replaced with a substituent such as alkyl, aryl, carboxylic acid, ester, amide, amine, urethane, ether, or carbonyl.

Component A of the polymeric backbone is derived from a substituted or unsubstituted polyol or mixture thereof, such as a diol, triol, tetraol or higher polyols. Suitable polyols include diols, such as alkane diols, preferably octanediol; triols, such as glycerol, trimethylolpropane, trimethylolpropane ethoxylate, triethanolamine; tetraols, such as erythritol, pentaerythritol; and higher polyols, such as sorbitol. Component A may also be derived from unsaturated polyols, such as tetradeca-2,12-diene-1,14-diol, polybutadiene-diol or other polyols including macromonomer polyols such as, for example polyethylene oxide, polycaprolactone triol and N-methyldiethanolamine (MDEA). Preferably, the polyol is substituted or unsubstituted glycerol.

Component B of the polymeric backbone is derived from a polyacid or mixture thereof, preferably a diacid or triacid. Exemplary acids include, but are not limited to, glutaric acid (5 carbons), adipic acid (6 carbons), pimelic acid (7 carbons), sebacic acid (8 carbons), azelaic acid (9 carbons) and citric acid. Exemplary long chain diacids include diacids having more than 10, more than 15, more than 20, and more than 25 carbon atoms. Non-aliphatic diacids can also be used. For example, versions of the above diacids having one or more double bonds can be used to produce polyol-diacid co-polymers. Preferably the polyacid is substituted or unsubstituted sebacic acid.

In an embodiment of the invention, the polymeric backbone is polyglycerol sebacate.

Polyol-based polymers described in US 8,912,304, US 7,722,894, US 8,143,042, US 2019/0071537 and US 2022/0380531, the contents of which are hereby incorporated by reference, are suitable pre-polymeric components for use in the present invention.

The molar ratios of the polyol to the polyacid in the polymeric backbone are suitably in the range of about 0.5:1 to about 1.5:1, preferably in the range of about 0.9:1.1 to about 1.1:0.9 and most preferably about 1:1.

Alternatively, the polymeric backbone of the pre-polymer is a polyamide or polyurethane backbone. For example, polyamine (comprising two or more amino groups) may be used to react with polyacid together with polyol or after reacting with polyol. Exemplary poly(ester amide) includes those described in Cheng et al., Adv. Mater. 2011, 23, 1195-11100, the contents of which are herein incorporated by reference. In other examples, polyisocyanates (comprising two or more isocyanate groups) may be used to react with polyacid together with polyol or after reacting with polyol. Exemplary polyester urethanes include those described in US 2013/231412.

In an embodiment, the pre-polymeric component in the composition of the invention contains activated groups on its polymeric backbone.

The activated groups are functional groups that can react or be reacted to form crosslinks. The pre-polymer is activated by reacting one or more functional groups on the monomer units of the backbone to provide one or more functional groups that can react or be reacted to form crosslinks resulting in cured polymer. Suitable functional groups to be activated on the pre-polymer backbone include hydroxy groups, carboxylic acid groups, amines, and combinations thereof, preferably hydroxy and/or carboxylic acid. The free hydroxy or carboxylic acid groups on the pre-polymer can be activated by functionalizing the hydroxy groups with a moiety which can form a crosslink between polymer chains. The groups that are activated can be free hydroxy or carboxylic acid groups on A and/or B moieties in the polymer backbone.

The free hydroxy or carboxylic groups can be functionalized with a variety of functional groups, for example vinyl groups. Vinyl groups can be introduced by a variety of techniques known in the art, such as by vinylation or acrylation. According to the present invention, vinyl groups contain the following structure -CRₚ=CR_{q}Rᵣ wherein Rₚ, R_{q}, Rᵣ are independently from one another, selected from the group consisting of H, alkyl such as methyl or ethyl, aryl such as phenyl, substituted alkyl, substituted aryl, carboxylic acid, ester, amide, amine, urethane, ether, and carbonyl.

Preferably, the activated group is or contains an acrylate group. According to the present invention, acrylate groups may contain the following group: -C(=O)-CRₓ=CR_{y}R_{z}, wherein Rₓ, R_{y}, R_{z} are independently from one another, selected from H, alkyl, alkenyl and aryl. The alkyl, alkenyl and aryl groups may be unsubstituted or substituted. According to an embodiment, the activated pre-polymer contains a mixture of different acrylate groups.

In an embodiment, the pre-polymeric component in the composition of the invention has functional (activated) groups on the polymeric backbone including functional groups of formula (I): 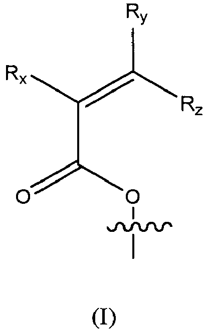 wherein Rₓ, R_{y} and R_{z} are independently selected from H, alkyl, alkenyl and aryl. The alkyl, alkenyl and aryl groups may be unsubstituted or substituted.

Preferably, all or part of the acrylate groups containing the --C(=O)-CRₓ=CR_{y}R_{z} group are such that Rₓ, R_{y} and R_{z} are H; or such that Rₓ is CH₃, R_{y} and R_{z} are H; or such that Rₓ and R_{y} are H and R_{z} is CH₃; or such that Rₓ and R_{y} are H and R_{z} is phenyl.

Functional (activated) groups of formula (I) can react or be reacted to form crosslinks.

In an embodiment of the invention, the pre-polymeric component is acrylated polyglycerol sebacate (PGSA). Synthetic routes to acrylated polyglycerol sebacate are described in WO 2016/202984.

In an embodiment of the invention, at least a proportion of the activated groups on the polymeric backbone of the pre-polymer may be acrylate groups (e.g. acrylate, methacrylate). The degree of activation (e.g. acrylation) is suitably measured by a technique such as ¹H NMR. The degree of activation (e.g. acrylation) is suitably characterized as "DA". The proportion of activated groups may be compared to the number of monomer units in the backbone. This can vary and can be from 0.1 to 0.8 mol/mol of monomer unit, preferably from 0.2 to 0.6 mol/mol of monomer unit and most preferably from 0.3 to 0.45 mol/mol of monomer unit, such as 0.3 mol/mol of monomer unit. It is most preferred when the degree of activation is as described above and the reactive functional group is acrylate i.e. degree of acrylation as above. When the polymeric unit of the backbone is of the general formula (-A-B-)ₙ, with A derived from a substituted or unsubstituted polyol and B derived from a substituted or unsubstituted polyacid, the monomer unit is of general formula -A-B- and the proportion of activated groups may be quoted per mole of polyacid or per mole of polyol. The DA ranges quoted above are preferably mol/mol of polyacid.

In an embodiment of the invention, the functional groups on the polymeric backbone include one or more functional groups that include a charged atom. The one or more functional groups that include a charged atom may be chosen from functional groups of formula (II), (III), (IV) and (V): 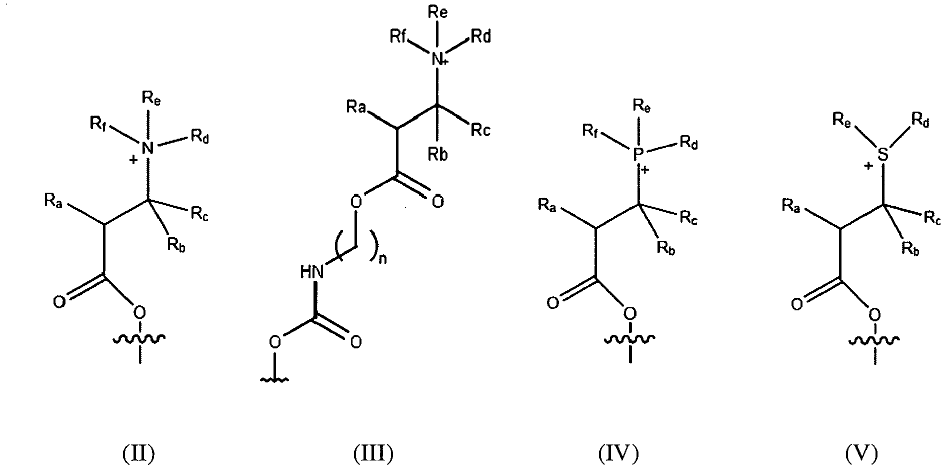 wherein Rₐ, R_{b}, R_{c}, R_{d}, Rₑ and R_{f} are independently selected from H, alkyl, alkenyl and aryl. The alkyl, alkenyl and aryl groups are unsubstituted or substituted. In an embodiment the invention, Rₐ, R_{b}, R_{c}, R_{d}, Rₑ and R_{f} are independently selected from H, alkyl, alkenyl and aryl wherein the alkyl, alkenyl and aryl groups are unsubstituted.

Suitable pre-polymeric components for use in the present invention are disclosed in WO 2021/078962 and WO 2022/180038. In an embodiment of the invention, the pre-polymeric component is acrylated and aminated polyglycerol sebacate (PGSAA) (i.e. activated and functionalized pre-polymeric component), e.g. a polymer of formula (VI): 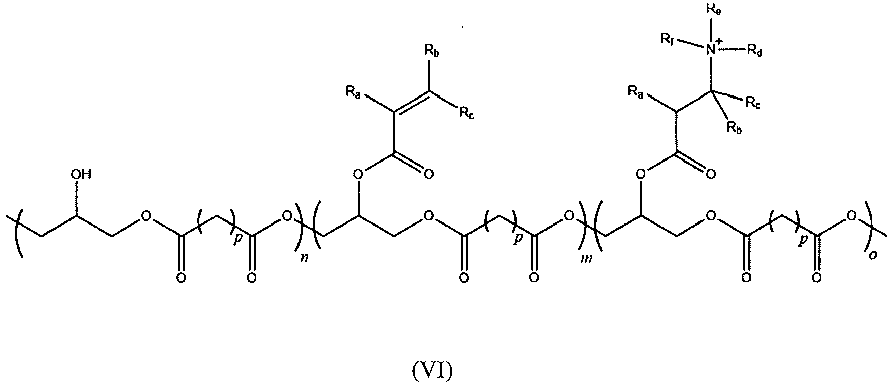 wherein p is between 1 and 20, wherein n, m and o are integers greater than 1, and wherein Ra, Rb, Re, Rd, Re and Rf are independently selected from H, alkyl, alkenyl and aryl.

Another representation of PGSAA is shown below: 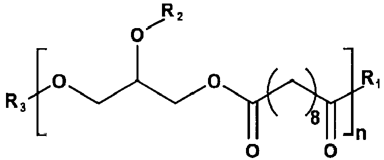
R₁ = OH, polymer chain, 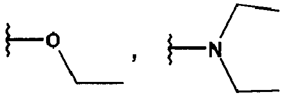
R₂ = H, polymer chain, 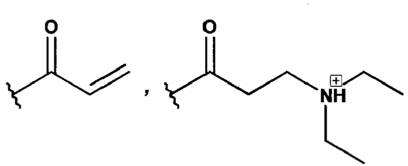
R₃ = H, **polymer** chain,

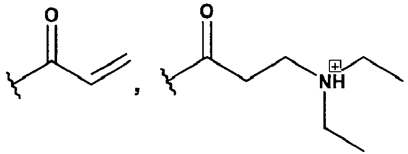

The weight average molecular weight of the pre-polymeric component (Mw), measured by Gel Permeation Chromatography equipped with a refractive index, may be from about 1,000 Daltons to about 1,000,000 Daltons, preferably from about 2,000 Daltons to about 500,000 Daltons, more preferably from about 2,000 Daltons to about 250,000 Daltons, most preferably from about 2,000 Daltons to about 100,000 Daltons. Advantageously the weight average molecular weight may be less than about 10,000 Daltons. The weight average molecular weight may be from about 1,000 Daltons to about 10,000 Daltons, from about 2,000 Daltons to about 10,000 Daltons, from about 3,000 Daltons to about 10,000 Daltons, from about 5,000 Daltons to about 10,000 Daltons. Preferably, it is about 4,500 Daltons.

The pre-polymeric component may have a polydispersity, measured by Gel Permeation Chromatography equipped with a refractive index, below 20.0, more preferably below 10.0, more preferably below 5.0, and even more preferably below 2.5. Preferably, it is about 2.5.

The term "about" as used herein means within 10%, preferably within 8%, and more preferably within 5% of a given value or range. According to a specific embodiment, "about X" means X, when X refers to the value or range.

### Porogen

A porogen is a component that dissolves and/or degrades *in vivo.* After the composition has been used to treat a fistula, the porogen will dissolve and/or degrade, resulting in a network of connected pores within the polymerized composition. The porogen is distinct from pre-polymeric component.

According to preferred embodiment, the polymerized composition is able to dissolve and/or degrade and the porogen dissolves and/or degrades faster than the polymerized pre-polymeric component.

In an embodiment of the invention, the porogen is a hydrogel. A hydrogel is a water-swollen polymeric material having a three-dimensional structure. The hydrogel comprises both a polymeric material and water.

If the porogen is a hydrogel, the weight ratio of polymeric material to water in the hydrogel is suitably in the range of 1:10 to 1:1, preferably from 1:6 to 1:2, more preferably from 1:4 to 1:3.

The polymeric material in the hydrogel may be a polypeptide such as collagen, gelatin, albumin or derivate thereof.

The polymeric material may be gelatin. The gelatin may be cross-linked such as cross-linked gelatin prepared by a thermal process. The gelatin may be modified gelatin, wherein functional groups have been grafted onto the gelatin, e.g. gelatin methacrylate. The gelatin is suitably medical-grade gelatin. Gelatin in the form of a powder may be used to prepare a hydrogel for use in the invention.

The polymeric material may be collagen.

The water in the hydrogel may be saline, such that the composition comprises salt (sodium chloride). The water is suitably physiological saline, having a salt concentration of about 0.9% w/v.

In another embodiment of the invention, the porogen is a sugar such as sucrose or trehalose, or a polysaccharide such as cellulose, alginate, chitosan or derivate thereof.

### Other Components

In an embodiment, the composition may comprise a photoinitiator.

Examples of suitable photoinitiators for use with UV light include, but are not limited to: 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl-1-phenyl-1-propanone (Darocur 1173), 2-benzyl-2-(dimehylamino)-1-[4-morpholinyl) phenyl]-1-butanone (Irgacure 369), methylbenzoylformate (Darocur MBF), oxy-phenyl-acetic acid-2-[2-oxo-2-phenyl-acetoxy-ethoxy]-ethyl ester (Irgacure 754), 2-methyl-1-[4-(methylthio)phenyl]-2-(4-morpholinyl)-1-propanone (Irgacure 907), diphenyl(2,4,6-trimethylbenzoyl)-phosphine oxide (Darocur TPO), phosphine oxide, phenyl bis(2,4,6-trimethyl benzoyl) (Irgacure 819), and combinations thereof.

Examples of photoinitiators for use with visible light include, but are not limited to, diphenyl(2,4,6-trimethylbenzoyl)-phosphine oxide, eosin Y disodium salt, N-Vinyl-2-Pyrrolidone (NVP) and triethanolamine, and camphorquinone.

In a preferred embodiment, the composition may comprise a redox system (i.e. a reducing agent and an oxidizing agent).

The reducing agent may be chosen from 4-N,N trimethylaniline, N,N-bis(2-hydroxyethyl)-p-toluidine, N,N-dimethylaniline, N,N-diethylaniline, sodium p-toluenesulfonate and N-methyl-N-(2-hydroxyethyl)-p-toluidine.

The oxidizing agent may be chosen from ammonium persulfate, potassium persulfate or benzoyl peroxide.

In one embodiment the redox system is 4-N,N trimethylaniline and benzoyl peroxide.

The reducing agent may be present in an amount from 0.1 to 5 wt%, based upon the weight of the composition. The oxidizing agent may be present in an amount from 0.1 to 10 wt%, based upon the weight of the composition.

In an embodiment an oxygen inhibitor, e.g., 4-(diphenylphosphino)styrene or triphenylphosphine, is incorporated in the composition with the redox system. The oxygen inhibitor may be present in an amount of less than 5 wt%, based upon the weight of the composition.

In an embodiment a radical scavenger, e.g., Tempo, Tempol or 4-methoxyphenol, is incorporated in the composition, for example in the composition comprising the redox system. The radical scavenger may be present in an amount from 0.005 to 0.5 wt%, based upon the weight of the composition.

According to a preferred embodiment, the radical scavenger is preferably 4-methoxyphenol.

According to an embodiment, the composition comprises a photoinitiator and a redox system (i.e. a reducing agent and an oxidizing agent).

The composition according to the invention may further contain one or more pharmaceutical, therapeutic, prophylactic, and/or diagnostic agents. The agent may be a small molecule agent, for example having molecular weight less than 2000, 1500, 1000, 750, or 500 Da, a biomolecule, for example peptide, protein, enzyme, nucleic acid, polysaccharide, growth factors, cell adhesion sequences such as RGD sequences or integrins, extracellular matrix components, or combinations thereof. Exemplary classes of small molecule agents include, but are not limited to, anti-inflammatories, analgesics, antimicrobial agents, and combinations thereof. Exemplary growth factors include, without limitation, TGF-β, acidic fibroblast growth factor, basic fibroblast growth factor, epidermal growth factor, IGF-I and II, vascular endothelial-derived growth factor, bone morphogenetic proteins, platelet-derived growth factor, heparin-binding growth factor, hematopoetic growth factor, peptide growth factor, or nucleic acids. Exemplary extracellular matrix components include, but are not limited to fibronectin, laminin, elastin and combinations thereof. These pharmaceutical, therapeutic, prophylactic, and/or diagnostic agents may be released at the fistula site once the composition has been delivered and cured.

The composition according to the invention may further comprise a coloring agent. Preferred examples of coloring agents are the ones recommended by the FDA for use in medical devices, pharmaceutical products or cosmetics.

### Composition properties

The pre-polymeric component may be present in an amount of from 20 to 60 wt%, based upon the weight of the composition, or may be present in amount of from 25 to 50 wt%, or from 30 to 45 wt%.

The porogen may be present in an amount of from 2 to 80 wt%, based upon the weight of the composition, or may be present in an amount of from 10 to 70 wt%, or from 30 to 65 wt%.

The amounts of the different components are adjusted by the skilled person to provide desirable properties for the composition, in particular viscosity. In an embodiment, the viscosity of the composition is such that composition is a gel at 25°C, having an elastic modulus G' greater than a viscous modulus G", when measured at 25°C. The viscosity of the composition is desirably such that it can be readily delivered into the fistula, and such that it will be retained within the fistula until the pre-polymer is cured.

According to a preferred embodiment:
- the pre-polymeric component is PGSAA and is present in an amount of from 25-50 wt%
- the porogen is sugar and is present in an amount of from 50-75 wt%
   or
- the porogen is gelatin hydrogel and is present in an amount of from 50-75wt% (this corresponds to dry gelatin is from 10-16wt%).

### Cross-linking of pre-polymeric component

The pre-polymeric component can undergo polymerization by cross-linking. Upon polymerization, the pre-polymer forms a crosslinked network that exhibits significant adhesive strength even in the presence of blood and other bodily fluids. The cured polymer obtained after curing is preferably sufficiently elastic to resist movement of the underlying tissue, for example tissue contractions. The adhesive cured polymer can provide a seal, preventing the leakage of fluids or gas. It is biodegradable and biocompatible, causing minimal inflammatory response.

The amounts of the different components in the composition may be adjusted to get an acceptable balance between adhesion and porosity (for tissue ingrowth). Higher amounts of the pre-polymeric component may lead to better adhesion, but may also lead to lower porosity, leading to inadequate tissue ingrowth.

The composition is applied to targeted localisation, e.g. to a fistula, before significant cross-linking of the pre-polymeric component, whilst the composition has a lower viscosity. After cross-linking, the composition becomes solid but not hard. After cross-linking, the composition becomes adhesive, ensuring that the composition is retained in place, e.g. within the fistula. After cross-linking, the composition is an adhesive and porous filler, is biocompatible, biodegradable, and can be colonized by cells.

In an embodiment, cross-linking is achieved by irradiation with light (e.g. UV light), in the presence of a photoinitiator to facilitate the reaction. The irradiation may take place as the composition is delivered, e.g. into the fistula. In one embodiment the composition may be delivered using a double-channel applicator, having one channel to deliver the composition and one channel wherein an optic fibre is positioned to irradiate the composition *in situ.*

In an embodiment, cross-linking is achieved by a redox reaction. A redox system (a reducing agent and an oxidizing agent) may be introduced into the composition such that a redox reaction takes place within the composition, leading to cross-linking.

Alternatively, it is further possible to achieve cross-linking both by light and by redox if the composition comprises both photoinitiator and redox system.

### Preparation of composition

The composition of the invention may be prepared by combining the pre-polymeric component and the porogen.

According to special embodiment, the pre-polymeric component is formulated with a radical scavenger, e.g., Tempo, Tempol or 4-methoxyphenol, before its combination with the porogen. According to a preferred embodiment, the radical scavenger is 4-methoxyphenol.

If the porogen is a hydrogel comprising a polypeptide, the composition of the invention may be prepared by steps of:
mixing water and the polypeptide to form a hydrogel; and
mixing the pre-polymeric component with the hydrogel.

According to a preferred embodiment, water is combined with salt (e.g. NaCl). According to preferred embodiment it is saline (also known as saline solution) which is a mixture of sodium chloride (salt) and water (0.9 w/v).

Alternatively, water might be replaced by solvents that are used in the medical space, e.g., DMSO, NMP, or mixtures thereof.

The composition is preferably prepared at room temperature (e.g. 25°C). Mixing may be carried out using any suitable method such as the use of a mixing syringe.

If the porogen is a sugar, the composition of the invention may be prepared by a step of:
mixing the sugar and the pre-polymeric component.

According to an embodiment, the porogen (including the hydrogel porogen) is combined with the photoinitiator or the reducing agent or the oxidizing agent before mixing with the pre-polymeric component. According to an embodiment, when the porogen (including the hydrogel porogen) is combined with the reducing agent or the oxidizing agent before the mixing step, the pre-polymeric component is combined with the oxidizing agent or the reducing agent, respectively (the redox process starts when reducing agent and oxidizing agent are mixed).

Suitably the composition of the invention is prepared immediately prior to use. This ensures that the composition has the required properties when delivered, e.g. into the fistula.

According to one non-limiting embodiment of the invention wherein the porogen is a hydrogel and the cross-linking is achieved by a redox reaction, the composition may be prepared in a method having steps as follows:
providing the polypeptide;
adding water, preferably saline, to the polypeptide and mixing the polypeptide and the water to form a hydrogel;
adding the pre-polymeric component to the hydrogel and mixing the pre-polymeric component and the hydrogel to form a hydrogel/pre-polymer mixture;
dividing the hydrogel/pre-polymer mixture into a first portion and a second portion;
mixing the first portion with an oxidizing agent to form an oxidizing mixture;
mixing the second portion with a reducing agent to form a reducing mixture; and
combining the oxidizing mixture and the reducing mixture.

### Treatment of tissue indications

The composition of the invention is used in indications where tissue reconstruction and tissue regrowth are necessary. "Tissue" intends to designate both hard tissues (such as bones or cartilages) or soft tissues. More particularly, the composition of the invention is used to the treatment of partial or full occlusion, augmentation, or filling of tissue lumens and voids. Lumens are taken to be the void space of tubular structures such as the vasculature, reproductive tract and gastro-intestinal tract. Voids are taken to include lesions, scars, fissures, fistulae and diverticulae. These voids can be physiological or the result of infection, surgery, cyst, tumor removal, or traumatic injury or remodeling of soft tissue. Specific examples are wound care, dental care, breast reconstruction, hernia prevention, osteoporosis.

While the invention is described below for use in treating fistulas, it is important to note that the composition of invention can similarly be used in treating any pathological situations where tissue reconstruction and tissue regrowth are necessary.

### Example of treatment: treatment of Fistulas

The composition of the invention may be used in the treatment of fistulas. In one embodiment of the invention, the fistula is a perianal fistula. Alternatively, the fistula may be an enterocutaneous fistula, a rectovaginal fistula or an oroantral fistula. The perianal fistula may be a simple fistula or a complex fistula.

The composition is delivered into the fistula. In one embodiment of the invention, the composition is delivered into the fistula using a syringe. The amount of composition that is delivered is suitably enough to fill the fistula.

The cross-linking reaction may be initiated immediately after the composition is delivered into the fistula, or whilst the composition is within the fistula. Upon polymerization, the composition becomes adhesive such that it adheres to the walls of the fistula. Preferably the cross-linking initiates immediately to 5 minutes of the composition being delivered into the fistula. Preferably the cross-linking will be complete within 30 sec to 30 minutes of the composition being delivered into the fistula, such that the composition rapidly becomes adhesive and is retained within the fistula.

The composition conforms to the fistula tract and is not extruded from the body during healing. The composition adheres to the fistula tissue. The porogen of the composition, when degrading or dissolving, generates pores throughout the cured composition that can be colonized by cells, enabling fistula healing. The pre-polymeric component is desirably biodegradable such that is degrades slowly inside the fistula. PGSA and PGSAA are biodegradable.

### Syringes and Kits

In an embodiment a syringe-based device is used to deliver the composition into soft tissue lumens and/or voids e.g. into a fistula.

A syringe may comprise the composition. Alternatively, a syringe may comprise one or more of the components of the composition, and the other components may be added to the syringe, prior to use.

For example, the syringe may comprise the porogen (optionally with the photoinitiator or reducing agent or oxidizing agent) and then the pre-polymeric component (optionally with the photoinitiator or reducing agent or oxidizing agent) may be added to the syringe prior to use. When a redox system is used, it is preferred that the reducing agent and the oxidizing agent are separated until the final mixing before use.

Suitable syringes may include a double barrel syringe and a syringe equipped with a mixing rod. Mixing of the components may occur within the syringe, e.g. using a mixing rod.

The invention also provides a kit of parts comprising, formulated separately from one another, (i) a first composition comprising a pre-polymeric component;
and (ii) a second composition comprising a porogen.

Prior to use, the first composition and the second composition may be mixed.

In an embodiment, a syringe has a first compartment and a second compartment, wherein the first compartment comprises a first composition comprising the pre-polymeric component, and wherein the second compartment comprises a second composition comprising the porogen.

In another embodiment, the kit of parts comprises a first syringe comprising the pre-polymeric component, and a second syringe comprising the porogen, the first and second syringes being connected with mixing devices (e.g. mixing rod or static mixer). A suitable device is described in US 9,662,676.

Prior to use, the first composition and the second composition may be mixed within the syringe, e.g. using a mixing rod.

According to an embodiment, the first composition and the second composition may be mixed in a third compartment.

According to a special embodiment, the first or second composition further comprises at least one photoinitiator and/or one agent of the redox system.

The kit of parts may also comprise instructions for use, e.g. for treating a fistula with the composition.

The invention will now be described with reference to examples which are not intending to be limiting of the invention and are provided as a guide to how the composition of the invention could be utilized to occlude, augment or fill soft tissue lumen and voids.

### EXAMPLES

### Preparation of Compositions

The components that were used in the compositions are summarized in Table 1 below:

**Table 1**

| Component | Description |
|---|---|
| Gelatin Type 1 | Formaldehyde-crosslinked gelatin from Gelita Medical - GelitaSpon GS-265 |
| Gelatin Type 2 | Heat-crosslinked gelatin from CuraMedical - CuraSpon CS-260 |
| Gelatin Type 3 | Gelatin that is not crosslinked from Weishardt - Gelatin G50 |
| Gelatin Type 4 | Heat-crosslinked gelatin from Mascia Brunelli - Cutanplast 05200001 |
| Collagen | Crosslinked collagen, bovine origin, from Symatese |
| Sucrose | Sucrose spheres (90-150µm) |
| PGSA | Acrylated polyglycerol sebacate produced by Tissium |
| PGSAA | Acrylated polyglycerol sebacate grafted with quaternary ammonium produced by Tissium (see WO2021078962) |
| Saline | From Gilbert Healthcare, 0.9% w/v NaCl in water |
| BPO Type A | Benzoyl peroxide from Arkema (Luperox A75), containing 75 wt% BPO and 25 wt% water, provided as a powder. |
| BPO Type B | Benzoyl peroxide from Arkema (Luperox A75), containing 75 wt% BPO and 25 wt% water, used dissolved in N-methyl pyrrolidone (250 mg/mL). |
| TMA | 4,N,N-trimethylaniline from Sigma Aldrich. |
| PPh₃ | Triphenylphosphine from TCI chemicals. Used dissolved in N-methyl-2-pyrrolidone (700 mg/mL). |
| DPPS | 4-(diphenylphosphino)styrene from Sigma Aldrich. Used dissolved in N-methyl-2-pyrrolidone (250 mg/mL). |
| NMP | N-methyl-2-pyrrolidone from VWR |
| TPO | diphenyl(2,4,6-trimethylbenzoyl)-phosphine oxide from Darocur |
| MEHQ | 4-methoxyphenol |

The compositions were extemporaneously prepared before use according to method well known in the art.

The formulations of the compositions are shown in Table 2 below:

**Table 2**

| Composition | Porogen | Pre-polymeric component | Other components | Curing type |
|---|---|---|---|---|
| 1 | 16.00 wt% Gelatin Type 1 | 25.00 wt% PGSAA (formulated with 5 000 ± 500 ppm TPO and 200 ± 50 ppm MEHQ) | | Photocuring |
| | 59.00 wt% Saline | | | |
| 2 | 15.20 wt% Gelatin Type 1 | 23.76 wt% PGSAA (formulated with 200 +/- 50 ppm MEHQ) | 2.50 wt% BPO | Redox |
| | | | Type A | |
| | | | 0.47 wt% | |
| | 56.06 wt% Saline | | TMA | |
| | | | 0.81 wt% PPh₃ | |
| | | | 1.20 wt% | |
| | | | NMP | |
| 3 | 16.00 wt% Gelatin Type 2 | 25.00 wt% PGSAA (formulated with 5 000 ± 500 ppm TPO and 200 ± 50 ppm MEHQ) | | Photocuring |
| | 59.00 wt% Saline | | | |
| 4 | 15.20 wt% Gelatin Type 2 | 23.76 wt% PGSAA (formulated with 200 +/- 50 ppm MEHQ) | 2.50 wt% BPO | Redox |
| | | | Type A | |
| | | | 0.47 wt% | |
| | 56.06 wt% Saline | | TMA | |
| | | | 0.81 wt% PPh₃ | |
| | | | 1.2 wt% NMP | |
| 5 | 16.00 wt% Gelatin Type 3 | 25.00 wt% PGSAA (formulated with 5 000 ± 500 ppm TPO and 200 ± 50 ppm MEHQ) | | Photocuring |
| | 59.00 wt% Saline | | | |
| 6 | 14.41 wt% Gelatin Type 3 | 22.52 wt% PGSAA (formulated with 200 +/- 50 ppm MEHQ) | 0.79 wt% BPO | Redox |
| | | | Type B | |
| | | | 0.44 wt% | |
| | 53.15 wt% Saline | | TMA | |
| | | | 0.85 wt% | |
| | | | DPPS | |
| | | | 7.83 wt% | |
| | | | NMP | |
| 7 | 14.50 wt% Gelatin Type 1 | 32.00 wt% PGSAA (formulated with 5 000 ± 500 ppm TPO and 200 ± 50 ppm MEHQ) | | Photocuring |
| | 53.50 wt% Saline | | | |
| 8 | 13.78 wt% Gelatin Type 1 | 30.41 wt% PGSAA (formulated with 200 +/- 50 ppm MEHQ) | 2.50 wt% BPO | Redox |
| | | | Type A | |
| | | | 0.47 wt% | |
| | 50.84 wt% Saline | | TMA | |
| | | | 0.81 wt% PPh₃ | |
| | | | 1.20 wt% | |
| | | | NMP | |
| 9 | 13.78 wt% Gelatin Type 4 | 30.41 wt% PGSAA (formulated with 200 +/- 50 ppm MEHQ) | 2.50 wt% BPO | Redox |
| | | | Type A | |
| | | | 0.47 wt% | |
| | 50.84 wt% Saline | | TMA | |
| | | | 0.81 wt% PPh₃ | |
| | | | 1.2 wt% NMP | |
| 10 | 13.78 wt% Gelatin Type 2 | 30.41 wt% PGSAA (formulated with 200 +/- 50 ppm MEHQ) | 2.50 wt% BPO | Redox |
| | | | Type A | |
| | | | 0.47 wt% | |
| | 50.84 wt% Saline | | TMA | |
| | | | 0.81 wt% PPh₃ | |
| | | | 1.2 wt% NMP | |
| 11 | 14.24 wt% Gelatin Type 2 | 31.43 wt% PGSAA (formulated with 200 +/- 50 ppm MEHQ) | 1.29 wt% BPO | Redox |
| | | | Type A | |
| | | | 0.48 wt% | |
| | 52.55 wt% Saline | | TMA | |
| 12 | 12.98 wt% Gelatin Type 2 | 37.00 wt% PGSAA (formulated with 200 +/- 50 ppm MEHQ) | 1.72 wt% BPO | Redox |
| | | | Type A | |
| | | | 0.38 wt% | |
| | 47.91 wt% Saline | | TMA | |
| 13 | 12.16 wt% Gelatin Type 2 | 38.01 wt% PGSAA (formulated with 200 +/- 50 ppm MEHQ) | 2.50 wt% BPO | Redox |
| | | | Type A | |
| | | | 0.47 wt% | |
| | 44.85 wt% Saline | | TMA | |
| | | | 0.81 wt% PPh₃ | |
| | | | 1.2 wt% NMP | |
| 14 | 12.53 wt% Gelatin Type 2 | 39.16 wt% PGSAA (formulated with 200 +/- 50 ppm MEHQ) | 1.72 wt% BPO | Redox |
| | | | Type A | |
| | | | 0.38 wt% | |
| | 46.20 wt% Saline | | TMA | |
| 15 | 10.17 wt% Gelatin Type 2 | 47.51 wt% PGSAA (formulated with 200 +/- 50 ppm MEHQ) | 2.50 wt% BPO | Redox |
| | | | Type A | |
| | | | 0.47 wt% | |
| | 37.34 wt% Saline | | TMA | |
| | | | 0.81 wt% PPh₃ | |
| | | | 1.2 wt% NMP | |
| 16 | 17.00 wt% Gelatin Type 1 | 20.00 wt% PGSAA (formulated with 5 000 + 500 ppm TPO and 200 ± 50 ppm MEHQ) | | Photocuring |
| | 63.00 wt% Saline | | | |
| 17 | 14.41 wt% Gelatin Type 1 | 22.52 wt% PGSAA (formulated with 200 +/- 50 ppm MEHQ) | 0.79 wt% BPO | Redox |
| | | | Type B | |
| | | | 0.44 wt% | |
| | 53.15 wt% Saline | | TMA | |
| | | | 0.85 wt% | |
| | | | DPPS | |
| | | | 7.83 wt% | |
| | | | NMP | |
| 18 | 70.00 wt% Sucrose | 30.00 wt% PGSAA (formulated with 5 000 ± 500 ppm TPO and 200 ± 50 ppm MEHQ) | | Photocuring |
| 19 | 9.00 wt% Collagen | 36.00 wt% PGSA (formulated with 5 000 ± 500 ppm TPO and 200 ± 50 ppm MEHQ) | | Photocuring |
| | 55.00 wt% Saline | | | |
| 20 | 9.00 wt% Gelatin Type 1 | 36.00 wt% PGSA (formulated with 5 000 ± 500 ppm TPO and 200 ± 50 ppm MEHQ) | | Photocuring |
| | 55.00 wt% Saline | | | |

### Adhesion Testing

Adhesion of the compositions following *in situ* curing was tested using a Lap Shear protocol having steps as follows:
- Porcine muscle tissue was cut to obtain rectangular samples.
- The product to be tested was deposited on one extremity of a muscle sample, as close as possible from the border. The other muscle sample was then deposited on top.
- Once curing was achieved the assembly was placed in the grips of a uniaxial mechanical tester vertically.
- Displacement and load were set to zero, then the upper grip went up at a rate of 5 mm/min until detachment of the two muscle samples. The load versus displacement curve was recorded and the maximum load (in N) before detachment was noted.
- The area of product was measured with a calliper at the end of the test.
- The maximum load (in N) was then divided by the area of polymer (cm²) in the overlap area. This is the apparent shear strength (N/cm²).

The results of the adhesion testing, including the number of times the test was repeated, are shown i n Table 3:

**Table 3**

| Composition | Repetitions | Adhesion (N/cm²) |
|---|---|---|
| 1 | 12 | 0.24 ± 0.06 |
| 2 | 28 | 0.26 ± 0.05 |
| 3 | 4 | 0.12 ± 0.01 |
| 4 | 16 | 0.14 ± 0.03 |
| 5 | 2 | 0.36 ± 0.10 |
| 6 | 4 | 0.43 ± 0.09 |
| 7 | 48 | 0.46 ± 0.19 |
| 8 | 12 | 0.40 ± 0.06 |
| 9 | 20 | 0.30 ± 0.11 |
| 10 | 16 | 0.38 ± 0.08 |
| 11 | 64 | 0.35 ± 0.15 |
| 12 | 4 | 0.53 ± 0.07 |
| 13 | 20 | 0.60 ± 0.20 |
| 14 | 176 | 0.60 ± 0.27 |
| 15 | 4 | 0.70 ± 0.26 |

### In vivo testing

The compositions were tested by subcutaneous implantation in rats and by treatment of perianal fistulas in pigs.

Cured discs of the compositions according to the invention were implanted into subcutaneous pockets in the back of rats for one and three months. The discs (h=2mm, d=5mm) were cured by light or by redox before implantation. The results are shown in Table 4:

**Table 4**

| Composition | Curing | Results |
|---|---|---|
| 16 | Photocuring | Cell invasion in the first hundreds of microns of the discs after one month (200-600µm) |
| 7 | Photocuring | Cell invasion in the first hundreds of microns of the discs after one month (70-300µm) |
| 17 | Redox | At one month: Cell invasion in the first hundreds of microns of the discs (70-500µm) |
| | | At three months: Full cell invasion of the discs. |
| 6 | Redox | At three months: Full cell invasion of the discs. |
| 10 | Redox | At one month: Full cell invasion of the discs. |
| 13 | Redox | At one month: Cell invasion in half of the discs. |
| 18 | Photocuring | At one month: Cell invasion throughout the discs. |
| 19 | Photocuring | At one month and three months: Cell invasion throughout the discs. Stronger inflammatory response compared to compositions with gelatin as porogen. |
| 20 | Photocuring | At one month and three months: Cell invasion throughout the discs |

For the porcine study, fistulas were created mechanically with catheters, dilators and drains in 9 animals. The drains were left in place for three weeks to allow creation of hollow fistula tracts (4 per animal). Each tract was debrided using a curette to remove granulation tissue and rinsed with saline. The internal opening was closed with two Vicryl 2-0 sutures in a X fashion. The composition of the invention was injected from the internal orifice (closed) to the external orifice in a retrograde manner, to fill the entire tract.

The study animals were sacrificed one month or three months after treatment.

Compositions 10, 11, 13 and 14 were tested. For all four compositions it was observed that the composition was not expelled from the fistula. Cell ingrowth (fibrous tissue penetrating the interstices of the polymer) was observed after one month and after three months. Minimal to mild mononuclear inflammatory cells and minimal to mild multinucleated foreign body-type giant cells were observed at the interface between the composition and the fibrous tissue and in the interstices of the implanted material.

## Claims

1. A composition for use in tissue reconstruction, wherein the composition comprises a pre-polymeric component and a porogen;
wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 12.

2. A composition for use according to claim 1, wherein there are functional groups on the polymeric backbone including functional groups of formula (I): 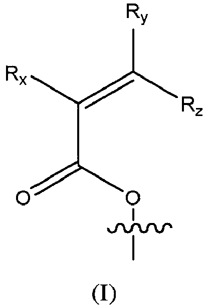 wherein Rₓ, R_{y} and R_{z} are independently selected from H, alkyl, alkenyl and aryl.

3. A composition for use according to any preceding claim, wherein the tissue reconstruction is treatment of a fistula, optionally a perianal fistula.

4. A composition for use according to any preceding claim, wherein the tissue reconstruction is treated by delivering the composition from a syringe.

5. A composition comprising a pre-polymeric component and a porogen;
wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 1, wherein there are functional groups on the polymeric backbone including functional groups of formula (I): 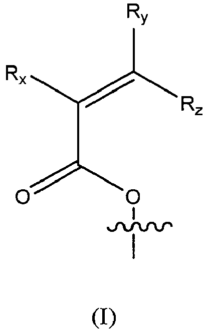
wherein Rₓ, R_{y} and R_{z} are independently selected from H, alkyl, alkenyl and aryl.

6. A composition for use according to claim 2, or a composition according to claim 5,
wherein the functional groups on the polymeric backbone further include one or more functional groups that include a charged atom.

7. A composition for use according to any one of claims 1 to 4 or 6, or a composition according to claim 5 or 6, wherein the porogen is a hydrogel, preferably comprising a polypeptide.

8. A composition for use according to claim 7, or a composition according to claim 7, wherein the polypeptide is gelatin.

9. A composition for use according to any one of claims 1 to 4 or 7 to 8, or a composition according to any one of claims 5 to 8, wherein the polyol is a triol, preferably glycerol or trimethylolpropane ethoxylate, and wherein the polyacid is a diacid selected from the group consisting of glutaric acid, adipic acid, pimelic acid, sebacic acid and azelaic acid, preferably sebacic acid.

10. A composition for use according to claim 9, or a composition according to claim 9, wherein the pre-polymeric component is selected from the group consisting of acrylated polyglycerol sebacate (PGSA) and acrylated and aminated polyglycerol sebacate (PGSAA).

11. A composition for use according to any one of claims 1 to 4 or 7 to 10, or a composition according to any one of claims 5 to 10, comprising a photoinitiator.

12. A composition for use according to any one of claims 1 to 4 or 7 to 10, or a composition according to any one of claims 5 to 10, comprising a redox system.

13. A syringe comprising a composition according to any one of claims 5 to 12.

14. A kit of parts comprising, formulated separately from one another,
(i) a first composition comprising a pre-polymeric component, wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 12; and
(ii) a second composition comprising a porogen.

15. A method for treating fistulas comprising delivering a composition into the fistula, wherein the composition comprises
a pre-polymeric component and a porogen;
wherein the polymeric backbone of the pre-polymeric component comprises a polymeric unit of general formula (-A-B-)ₙ, wherein A is derived from a substituted or unsubstituted polyol and B is derived from a substituted or unsubstituted polyacid, and n is greater than 1.
